# EUROPÄISCHE PATENTSCHRIFT

(11) **EP 3 233 768 B1**
(45) Veröffentlichungstag und Bekanntmachung des Hinweises auf die Patenterteilung: **20.02.2019**
(21) Anmeldenummer: 15813771.1
(22) Anmeldetag: 15.12.2015
(51) Int. Cl.: C07C 2/02, C07C 11/167, B01J 31/22, B01J 31/24

(54) **1,3-BUTADIEN-SYNTHESE**
1,3-BUTADIENE SYNTHESIS
SYNTHÈSE DE 1,3-BUTADIÈNE

(30) Priorität: 16.12.2014 EP 14198129
(43) Veröffentlichungstag der Anmeldung: 25.10.2017
(73) Patentinhaber: ARLANXEO Deutschland GmbH, 41540 Dormagen (DE)
(72) Erfinder: DREISBACH, Claus, 42799 Leichlingen (DE); SCHLENK, Stefan, 86916 Kaufering (DE); HOFFMANN, Martina, 51469 Bergisch Gladbach (DE); LARCHER, Christoph, 40476 Düsseldorf (DE); FÖLLINGER, Thomas, 41539 Dormagen (DE)
(74) Vertreter: Wichmann, Birgid
(86) Internationale Anmeldenummer: PCT/EP2015/079778
(87) Internationale Veröffentlichungsnummer: WO 2016/096846

(56) Entgegenhaltungen:
- US-A- 2 377 025
- US-A- 2 420 477
- IOAN-TEODOR TROTUS ET AL: "Butadiene from acetylene-ethylene cross-metathesis", CHEMICAL COMMUNICATIONS, Bd. 51, Nr. 33, 1. Januar 2015 (2015-01-01), Seiten 7124-7127, XP055189505, ISSN: 1359-7345, DOI: 10.1039/C5CC00853K

## Beschreibung

Die Erfindung betrifft ein Verfahren zur Herstellung von 1,3-Butadien mittels En-In-Metathese an wenigstens einem Übergangsmetallkatalysator des Elementes Ruthenium.

1,3-Butadien (CAS Nr. 106-99-0) wird zumeist durch thermische Spaltung (Steam-Cracken) gesättigter Kohlenwasserstoffe hergestellt, wobei üblicherweise von Naphtha oder Kohlenwasserstoffen im Bereich C2 bis C4 als Rohstoff ausgegangen wird. Beim Aufarbeiten des beim Cracken gebildeten Stoffgemisches fällt ein sogenannter C4-Schnitt an, der neben 1,3-Butadien auch 1,2-Butadien, n-Butan, Butene, 1-Butin, Butenin und Propin enthält.

Nachteilig an der Gewinnung von 1,3-Butadien aus Crackerprodukten ist, dass als Crackerrohstoff zu Lasten des Naphtha immer mehr Ethan eingesetzt wird. Das Cracken von Ethan liefert aber nur minimale Mengen an 1,3-Butadien, so dass dieses stetig knapper und teurer wird. Ein Hauptprodukt aus Crackprozessen ist zudem aber auch Ethen.

Ein weiteres wichtiges Edukt in der großtechnischen Herstellung wichtiger Grundchemikalien ist Acetylen, auch als Ethin bezeichnet, das großtechnisch mittels Hochtemperaturpyrolyse von leichten oder mittleren Erdölfraktionen oder Erdgas bei 2.000 °C hergestellt wird. Nach der Pyrolyse wird das entstandene Gasgemisch schnell unter 200 °C abgekühlt (*gequencht*), um die Zersetzung des Produkts zu elementarem Kohlenstoff und Wasserstoff zu vermeiden.

Aus US-A 3,671,604 ist die katalytische Copolymerisation von Ethin mit Monoolefinen bekannt, wobei saure Katalysatoren aus der Gruppe Zinkacetat, Zinkoxid, Cadmiumoxid, Cadmiumacetat, Nickeloxid, Nickelacetat, Cobaltoxid und Cobaltacetat eingesetzt werden. Die im Verfahren der US-A 3,671,604 getesteten Reaktionstemperaturen lagen im Bereich von 150 °C bis 325 °C, wobei nur Temperaturen im Bereich von 250 °C bis 300 °C zu messbaren Produktausbeuten führten. Das Reaktionsprodukt war ein Gemisch aus dreißig bis vierzig Komponenten.

US 2,377,025 beschreibt ein Verfahren zur Herstellung von 1,3-Butadien durch Umsetzung von Acetaldehyd zu Ethin und anschließende Reaktion des Ethins mit Ethan. Beide Schritte erfolgen in Gegenwart eines heterogenen Katalysators, der ein dehydrierendes Metall, wie etwa Kupfer, Silber oder Quecksilber sowie weitere Metalle umfasst.

Ausgehend von diesem Stand der Technik bestand die Aufgabe der vorliegenden Erfindung darin, ein Verfahren zur Herstellung gezielt von 1,3-Butadien aus den technisch in großen Mengen zur Verfügung stehenden Edukten Ethin und Ethen bereit - zu stellen, das zudem bei deutlich niedrigeren Temperaturen als der Stand der Technik abläuft.

Gelöst wird die Aufgabe durch ein Verfahren zur Herstellung von 1,3-Butadien indem man Ethen und Ethin in Gegenwart mindestens eines homogenen Übergangsmetallkatalysators des Elementes Ruthenium miteinander umsetzt. Hierbei handelt es sich um eine En-In-Metathese.
Zur Klarstellung sei angemerkt, dass vom Rahmen dieser Erfindung alle nachfolgend auf-geführten allgemeinen oder in Vorzugsbereichen genannten Definitionen und Parameter in beliebigen Kombinationen umfasst sind.

Die Erfindung betrifft ferner die Verwendung von homogenen Übergangsmetallkatalysatoren des Elementes Ruthenium zur Herstellung von 1,3-Butadien aus Ethin und Ethen.
Merkmal einer homogenen Katalyse ist, dass die Reaktanden bzw. Edukte, die Produkte und der Katalysator unter Reaktionsbedingungen in einer einzigen Phase im Sinne der Gibbsschen Phasenregel vorliegen. Bei dieser Phase kann es sich um ein Gas, eine Flüssigkeit oder ein überkritisches Fluid handeln. Die mit Abstand bedeutendsten Katalysatoren in der homogenen Katalyse und deshalb erfindungsgemäß bevorzugt sind metallorganische Verbindungen molekular gelöst in einer flüssigen Phase.
Bevorzugt wird deshalb die erfindungsgemäße 1,3-Butadien Synthese durch Umsetzung von Ethen und Ethin in flüssiger Phase in Anwesenheit wenigstens eines homogenen Übergangsmetallkatalysators des Elementes Ruthenium durchgeführt. Hierbei wird der wenigstens eine Übergangsmetallkatalysator des Elementes Ruthenium in Lösung eingesetzt. Für das Verfahren geeignete Lösungsmittel lösen den homogenen Katalysator, aber zersetzen diesen nicht. Bevorzugt wird als Lösungsmittel wenigstens eines aus der Gruppe der bei Raumtemperatur flüssigen Halogenalkane eingesetzt, wobei Raumtemperatur 23 +/- 2 °C bedeutet. Insbesondere bevorzugt ist Dichlormethan.
Die Reaktionstemperatur des erfindungsgemäßen Verfahrens ist abhängig von der Zersetzungstemperatur des einzusetzenden Katalysators. Sie kann vom Fachmann durch übliche Überlegungen gewählt werden. In bevorzugter Ausführungsform erfolgt die erfindungsgemäße 1,3-Butadien Synthese deshalb bei Temperaturen im Bereich von - 70 bis 50 °C, besonders bevorzugt im Bereich von - 64 bis 45 °C, ganz besonders bevorzugt im Bereich von - 50 bis 40 °C, insbesondere bevorzugt im Bereich von 15 bis 40 °C. Aufgrund dieser niedrigen Temperaturen im Vergleich zum oben zitierten Stand der Technik ist das erfindungsgemäße Verfahren deutlich wirtschaftlicher.

Die erfindungsgemäße 1,3-Butadien Synthese wird vorzugsweise ohne Überdruck durchgeführt. Das heißt, dass in bevorzugter Ausführungsform das erfindungsgemäße Verfahren bei Normaldruck von 1013,25 hPa +/- 5 % durchgeführt wird.

Die erfindungsgemäße Synthese von 1,3-Butadien kann sowohl absatzweise (batch-Verfahren) als auch kontinuierlich durchgeführt werden. Vorzugsweise wird die erfindungsgemäße 1,3-Butadien Synthese batchweise durchgeführt. Bei der batchweisen Durchführung liegen die bevorzugten Reaktionszeiten im Bereich von 0,5 bis 4 Stunden, wobei die Reaktionszeit definiert wird als die Gesamtzeit, während der die Reaktionsgase eingeleitet werden.

Oftmals sind homogene Katalysatoren ausgesprochen oxidations- und/oder hydrolyseempfindlich, so dass in bevorzugter Ausführungsform des erfindungsgemäßen Verfahrens dieses unter anaeroben Bedingungen durchgeführt wird, bevorzugt unter Schutzgas. Bevorzugt werden als Schutzgas Stickstoff oder Edelgase eingesetzt, insbesondere Stickstoff.

Die meisten metallorganischen Komplexkatalysatoren sind Übergangsmetallverbindungen mit definierter Struktur und Stöchiometrie. Bei gut untersuchten Prozessen lässt sich der Reaktionsablauf auf molekularer Basis (nahezu) vollständig verstehen, was Grundlage für eine gezielte Entwicklung von Katalysatoren nach Maß ist. Typischerweise ist das Reaktionszentrum ein Metallatom (-ion), an dem auch Liganden koordiniert sind, die nicht direkt an der Katalyse beteiligt sind (spectator ligands, control ligands). Variationen dieser Liganden unter dem Gesichtspunkt, gezielt die elektronischen und/oder sterischen Verhältnisse am Reaktionszentrum zu beeinflussen (ligand tuning), sind Grundlage für Katalysatoroptimierungen hinsichtlich Aktivität, Selektivität und Stabilität.

Erfindungsgemäß bevorzugt sind deshalb homogene Übergangsmetallkatalysatoren des Elementes Ruthenium mit wenigstens einem organischen Ligand.

Als erfindungsgemäß einzusetzende Übergangsmetallkatalysatoren werden bevorzugt solche der allgemeinen Formel (A) eingesetzt, 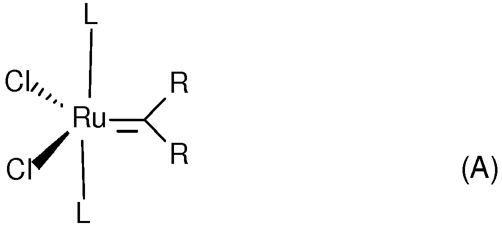 worin
- L: gleiche oder verschiedene Liganden, bevorzugt neutrale Elektronen-Donoren darstellen, oder L ist ein Sauerstoffatom eines Substituenten R ist, das koordinativ am Ruthenium gebunden ist, und
- R: gleich oder verschieden sind und Wasserstoff, Alkyl, Cycloalkyl, Alkenyl, Alkinyl, Aryl, Carboxylat, Alkoxy, Alkenyloxy, Alkinyloxy, Aryloxy, Alkoxycarbonyl, Alkylamino, Alkylthio, Arylthio, Alkylsulfonyl oder Alkylsulfinyl, bedeuten wobei diese Reste alle jeweils optional durch ein oder mehrere Alkyl-, Halogen-, Alkoxy-, Aryl- oder Heteroaryl-Reste substituiert sein können, oder alternativ beide Reste R unter Einbindung des gemeinsamen C-Atoms, an das sie gebunden sind, zu einer cyclischen Gruppe verbrückt sind, die aliphatischer oder aromatischer Natur sein kann, gegebenenfalls substituiert ist und ein oder mehrere Heteroatome enthalten kann.

Bevorzugt steht R in der allgemeinen Formel (A) jeweils unabhängig voneinander für Wasserstoff, C₁-C₃₀-Alkyl, C₃-C₂₀-Cycloalkyl, C₂-C₂₀-Alkenyl, C₂-C₂₀-Alkinyl, C₆-C₂₄-Aryl, C₁-C₂₀-Carboxylat, C₁-C₂₀-Alkoxy, C₂-C₂₀-Alkenyloxy, C₂-C₂₀-Alkinyloxy, C₆-C₂₄-Aryloxy, C₂-C₂₀-Alkoxycarbonyl, C₁-C₃₀-Alkylamino, C₁-C₃₀-Alkylthio, C₆-C₂₄-Arylthio, C₁-C₂₀-Alkylsulfonyl oder C₁-C₂₀-Alkylsulfinyl, wobei diese Reste alle jeweils optional durch ein oder mehrere C₁-C₆-Alkyl-, Halogen-, C₁-C₆-Alkoxy-, bevorzugt i-Propoxy-, Aryl-, oder Heteroaryl-Reste substituiert sein können, oder alternativ beide Reste R unter Einbindung des gemeinsamen C-Atoms, an das sie gebunden sind, zu einer cyclischen Gruppe verbrückt sind, die aliphatischer oder aromatischer Natur sein kann, gegebenenfalls substituiert ist und ein oder mehrere Heteroatome enthalten kann.

In bevorzugter Ausführungsform können die beiden Liganden L unabhängig voneinander einen Phosphin-, sulfonierten Phosphin-, Phosphat-, Phosphinit-, Phosphonit-, Ether-, Amin-, Amid-, Sulfoxid-, Carboxyl-, Nitrosyl-, Pyridin-, Thioether- oder einen Imidazolidin ("Im")-Liganden darstellen.

Bevorzugt bedeuten die beiden Liganden L unabhängig voneinander einen C₆-C₂₄-Aryl-, C₁-C₁₀-Alkyl- oder C₃-C₂₀-Cycloalkyl-Phosphin-Liganden, einen sulfonierten C₆-C₂₄-Aryl- oder sulfonierten C₁-C₁₀-Alkyl-Phosphin-Liganden, einen C₆-C₂₄-Aryl- oder C₁-C₁₀-Alkyl-Phosphinit-Liganden, einen C₆-C₂₄-Aryl- oder C₁-C₁₀-Alkylphosphonit-Liganden, einen C₆-C₂₄-Aryl- oder C₁-C₁₀-Alkylphosphit-Liganden, einen C₆-C₂₄-Aryl- oder C₁-C₁₀-Alkylarsin-Liganden, einen C₆-C₂₄-Aryl- oder C₁-C₁₀-Alkylamin-Liganden, einen Pyridin-Liganden, einen C₆-C₂₄-Aryl- oder C₁-C₁₀-Alkyl-Sulfoxid-Liganden, einen C₆-C₂₄-Aryl- oder C₁-C₁₀-Alkyl-Ether-Liganden oder einen C₆-C₂₄-Aryl- oder C₁-C₁₀-Alkylamid-Liganden, die alle jeweils durch eine Phenylgruppe substituiert sein können, die wiederum gegebenenfalls durch einen Halogen-, C₁-C₅-Alkyl- oder C₁-C₅-Alkoxy-Rest substituiert ist, oder L ist ein Sauerstoffatom eines Substituenten R, das koordinativ am Ruthenium gebunden ist.

Die Bezeichnung "Phosphin" schließt insbesondere PPh₃, P(p-Tol)₃, P(o-Tol)₃, PPh(CH₃)₂, P(CF₃)₃, P(p-FC₆H₄)₃, P(p-CF₃C₆H₄)₃, P(C₆H₄-SO₃Na)₃, P(CH₂C₆H₄-SO₃Na)₃, P(iso-Propyl)₃, P(CHCH₃(CH₂CH₃))₃, P(Cyclopentyl)₃, P(Cyclohexyl)₃, P(Neopentyl)₃ und P(Neophenyl)₃ ein.

"Alkyl" bezeichnet im Rahmen der vorliegenden Erfindung eine geradkettige oder verzweigte gesättigte Kohlenwasserstoffgruppe. In einigen Ausführungsformen wird eine Alkylgruppe mit 1 bis 6 Kohlenstoffatomen eingesetzt und kann als eine "Niederalkylgruppe" bezeichnet werden. Bevorzugte Alkylgruppen sind Methyl (Me), Ethyl (Et), Propyl, insbesondere n-Propyl und iso-Propyl, Butyl, insbesondere n-Butyl, iso-Butyl, sec-Butyl, tert-Butyl, Pentyl-Gruppen, insbesondere n-Pentyl, iso-Pentyl, neo-Pentyl, Hexylgruppen und dergleichen.

"Aryl" bezeichnet im Rahmen der vorliegenden Erfindung ein monocyclisches aromatisches Kohlenwasserstoff-Ringsystem oder ein polycyclisches Ringsystem, in dem zwei oder mehrere aromatische Kohlenwasserstoff-Ringe kondensiert sind, oder wenigstens einen aromatischen monocyclischen Kohlenwasserstoffring, der mit einem oder mehreren Cycloalkyl- und / oder Cycloheteroalkylringen fusioniert ist. Eine Arylgruppe kann 6 bis 24 Kohlenstoffatome in seinem Ringsystem aufweisen, beispielsweise eine C₆₋₂₀ Arylgruppe, und dabei mehrere kondensierte Ringe enthalten. In einigen Ausführungsformen kann Aryl oder Arylen eine polycyclische Arylgruppe mit 8 bis 24 Kohlenstoffatomen bedeuten. Bevorzugte Arylgruppen mit einem aromatischen carbocyclischen Ringsystem sind Phenyl, 1-Naphthyl (bicyclisch), 2-Naphthyl (bicyclisch), Anthracenyl (tricyclisch), Phenanthrenyl (tricyclisch) Pentacenyl (penta) und ähnliche Gruppen. Andere bevorzugte Arylgruppen sind Benzodioxanyl, Benzodioxolyl, Chromanyl, Indolinyl-Gruppen und dergleichen. In einigen Ausführungsformen können Arylgruppen, insbesondere Phenylgruppen, wie hierin beschrieben, substituiert sein, insbesondere durch einen i-Propoxyrest. In einigen Ausführungsformen kann eine Arylgruppe einen oder mehrere Substituenten aufweisen. Insbesondere bevorzugt steht Aryl für Phenyl.

"Cycloalkyl" im Sinne der vorliegenden Erfindung bezeichnet eine nicht-aromatische carbocyclische Gruppe, die cyclisierte Alkyl, Alkenyl oder Alkinyl-Gruppen enthält. In verschiedenen Ausführungsformen enthält eine Cycloalkylgruppe 3 bis 24 Kohlenstoffatome, bevorzugt 3 bis 20 Kohlenstoffatome, z.B eine C₃-₁₄ Cycloalkylgruppe. Eine Cycloalkylgruppe kann monocyclisch, wie beispielsweise Cyclohexyl, oder aber auch polycyclisch sein, wie beispielsweise in überbrückten und / oder Spiro-Ringsystemen, wobei die Kohlenstoffatome innerhalb oder außerhalb des Ringsystems sich befinden können. Jede geeignete Ringposition der Cycloalkylgruppe kann kovalent an die definierte chemische Struktur gebunden sein. Bevorzugte Cycloalkylgruppen sind Cyclopropyl, Cyclobutyl, Cyclopentyl, Cyclohexyl, Cycloheptyl, Cyclopentenyl, Cyclohexenyl, Cyclohexadienyl, Cycloheptatrienyl, Norbornyl, Norpinyl, Norcaryl, Adamantyl und Spiro [4.5] decanyl-Gruppen sowie deren Homologe, Isomere und dergleichen. In einigen Ausführungsformen können die Cycloalkylgruppen substituiert sein. Erfindungsgemäß bevorzugt sind unsubstituierte Cycloalkylgruppen.

"Arylalkyl" im Sinne der vorliegenden Erfindung bedeutet eine Alkyl-Aryl-Gruppe, wobei die Arylalkyl-Gruppe kovalent an die definierte chemische Struktur durch die Alkylgruppe gebunden ist. Eine erfindungsgemäß bevorzugte Arylalkylgruppe ist die Benzyl-Gruppe (-CH₂-C₆H₅). Arylalkylgruppen gemäß der vorliegenden Erfindung können wahlweise substituiert sein, das heißt, entweder die Arylgruppe und / oder die Alkyl-Gruppe kann substituiert sein.

Die Bezeichnung "Phosphinit" schließt beispielsweise Triphenylphosphinit, Tricyclohexylphosphinit, Triisopropylphosphinit und Methyldiphenylphosphinit ein.

Die Bezeichnung "Phosphit" schließt insbesondere Triphenylphosphit, Tricyclohexylphosphit, Tri-tert.-Butylphosphit, Triisopropylphosphit und Methyldiphenylphosphit ein.

Die Bezeichnung "Sulfonat" schließt insbesondere Trifluoromethansulfonat, Tosylat und Mesylat ein.

Die Bezeichnung "Sulfoxid" schließt insbesondere (CH₃)₂S(=O) und (C₆H₅)₂S=O ein.

Die Bezeichnung "Thioether" schließt insbesondere CH₃SCH₃, C₆H₅SCH₃, CH₃OCH₂CH₂SCH₃ und Tetrahydrothiophen ein.

Die Bezeichnung "Pyridin" soll im Rahmen dieser Anmeldung als Oberbegriff sämtliche stickstoffhaltigen Liganden einschließen wie sie z.B. von Grubbs in der WO-A-03/011455 genannt werden. Beispiele hierfür sind: Pyridin, Picoline (α-, β-, und γ-Picolin), Lutidine (2,3-, 2,4-, 2,5-, 2,6-, 3,4- und 3,5-Lutidin), Collidin (2,4,6-Trimethylpyridin), Trifluormethylpyridin, Phenylpyridin, 4-(Dimethylamino)pyridin, Chlorpyridine, Brompyridine, Nitropyridine, Chinolin, Pyrimidin, Pyrrol, Imidazol und Phenylimidazol.

Handelt es sich bei einem oder beiden der Liganden L in Formel (A) um einen Imidazolidinrest (Im), so weist dieser üblicherweise eine Struktur der allgemeinen Formeln (IIa) oder (IIb) auf, 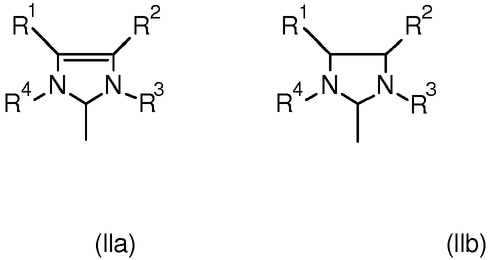 worin
R¹, R², R³, R⁴ gleich oder verschieden sind und Wasserstoff, geradkettiges oder verzweigtes C₁-C₃₀-Alkyl, C₃-C₂₀-Cycloalkyl, C₂-C₂₀-Alkenyl, C₂-C₂₀-Alkinyl, C₆-C₂₄-Aryl, C₁-C₂₀-Carboxylat, C₁-C₂₀-Alkoxy, C₂-C₂₀-Alkenyloxy, C₂-C₂₀-Alkinyloxy, C₆-C₂₀-Aryloxy, C₂-C₂₀-Alkoxycarbonyl, C₁-C₂₀-Alkylthio, C₆-C₂₀-Arylthio, C₁-C₂₀-Alkylsulfonyl, C₁-C₂₀-Alkylsulfonat, C₆-C₂₀-Arylsulfonat oder C₁-C₂₀-Alkylsulfinyl bedeuten.

Gegebenenfalls kann einer oder mehrere der Reste R¹, R², R³, R⁴ unabhängig voneinander durch einen oder mehrere Substituenten, vorzugsweise geradkettiges oder verzweigtes C₁-C₁₀-Alkyl, C₃-C₈-Cycloalkyl, C₁-C₁₀-Alkoxy oder C₆-C₂₄-Aryl substituiert sein, wobei diese vorgenannten Substituenten wiederum durch ein oder mehrere Reste, vorzugsweise ausgewählt aus der Gruppe Halogen, insbesondere Chlor oder Brom, C₁-C₅-Alkyl, C₁-C₅-Alkoxy und Phenyl substituiert sein können.

Nur zur Klarstellung sei hinzugefügt, dass die in den allgemeinen Formeln (IIa) und (IIb) dargestellten Strukturen des Imidazolidinrests mit den in der Literatur für diesen Imidazolidinrest (Im) dargestellten Strukturen gleichbedeutend sind, die den Carben-Charakter des Imidazolidinrests hervorheben. Dies gilt entsprechend auch für die zugehörigen bevorzugten, nachfolgend noch dargestellten Strukturen (IIIa)-(IIIf).

In einer bevorzugten Ausführungsform der Katalysatoren der allgemeinen Formel (A) mit wenigstens einem Imidazolidinrest (Im) als Liganden L bedeuten R¹ und R² unabhängig voneinander Wasserstoff, C₆-C₂₄-Aryl, besonders bevorzugt Phenyl, geradkettiges oder verzweigtes C₁-C₁₀-Alkyl, besonders bevorzugt Propyl oder Butyl, oder bilden zusammen unter Einschluss der Kohlenstoffatome, an die sie gebunden sind, einen Cycloalkyl- oder Aryl-Rest, wobei alle vorgenannten Reste gegebenenfalls wiederum durch ein oder mehrere weitere Reste substituiert sein können, ausgewählt aus der Gruppe umfassend geradkettiges oder verzweigtes C₁-C₁₀-Alkyl, C₁-C₁₀-Alkoxy, C₆-C₂₄-Aryl und eine funktionelle Gruppe ausgewählt aus der Gruppe von Hydroxy, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat und Halogen.

In einer bevorzugten Ausführungsform der Katalysatoren der allgemeinen Formel (A) mit wenigstens einem Imidazolidinrest (Im) als Liganden L sind ferner die Reste R³ und R⁴ gleich oder verschieden und bedeuten geradkettiges oder verzweigtes C₁-C₁₀-Alkyl, besonders bevorzugt i-Propyl oder Neopentyl, C₃-C₁₀-Cycloalkyl, bevorzugt Adamantyl, C₆-C₂₄-Aryl, besonders bevorzugt Phenyl, C₁-C₁₀-Alkylsulfonat, besonders bevorzugt Methansulfonat, C₆-C₁₀-Arylsulfonat, besonders bevorzugt p-Toluolsulfonat.

Gegebenenfalls sind die vorgenannten Reste als Bedeutungen von R³ und R⁴ substituiert durch einen oder mehrere weitere Reste ausgewählt aus der Gruppe umfassend geradkettiges oder verzweigtes C₁-C₅-Alkyl, insbesondere Methyl, C₁-C₅-Alkoxy, Aryl und eine funktionelle Gruppe ausgewählt aus der Gruppe von Hydroxy, Thiol, Thioether, Keton, Aldehyd, Ester, Ether, Amin, Imin, Amid, Nitro, Carbonsäure, Disulfid, Carbonat, Isocyanat, Carbodiimid, Carboalkoxy, Carbamat und Halogen.

Insbesondere können die Reste R³ und R⁴ gleich oder verschieden sein und bedeuten i-Propyl, Neopentyl, Adamantyl, Mesityl oder 2,6-Diisopropylphenyl.

Besonders bevorzugte Imidazolidinreste (Im) im Liganden L haben die nachfolgenden Strukturen (IIIa) bis (IIIf), wobei Ph jeweils für einen Phenyl-Rest, Bu für einen Butyl-Rest und Mes jeweils für 2,4,6-Trimethylphenyl-Rest steht oder Mes alternativ in allen Fällen für 2,6-Diisopropylphenyl steht.

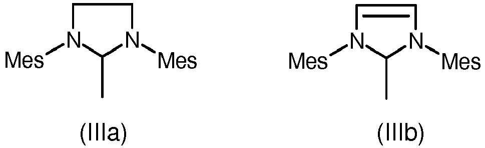

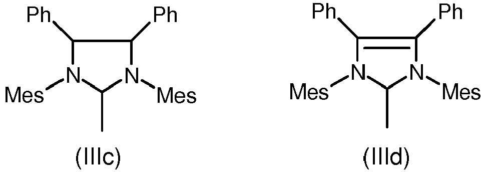

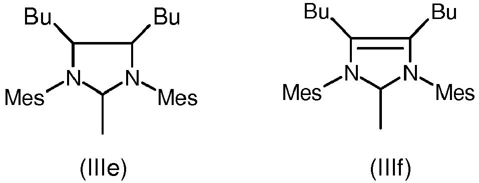

Verschiedenste Vertreter der Katalysatoren der Formel (A) sind prinzipiell bekannt, so z.B. aus der WO-A-96/04289 und der WO-A-97/06185.

Alternativ zu den bevorzugten Im-Resten steht einer oder beide Liganden L in der allgemeinen Formel (A) bevorzugt auch für gleiche oder verschiedene Trialkylphosphin-Liganden, worin mindestens eine der Alkylgruppen eine sekundäre Alkylgruppe oder eine Cycloalkylgruppe darstellt, bevorzugt iso-Propyl, iso-Butyl, sec-Butyl, Neopentyl, Cyclopentyl oder Cyclohexyl.

Besonders bevorzugt stehen in der allgemeinen Formel (A) einer oder beide Liganden L für einen Trialkylphosphin-Liganden, worin mindestens eine der Alkylgruppen eine sekundäre Alkylgruppe oder eine Cycloalkylgruppe darstellt, bevorzugt iso-Propyl, iso-Butyl, sec-Butyl, Neopentyl, Cyclopentyl oder Cyclohexyl.

In einer bevorzugten Ausführungsform werden Ruthenium basierte Übergangsmetallkatalysatoren der Formel (B) eingesetzt, 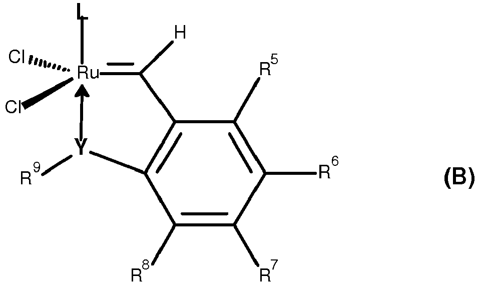 worin
- Y: Sauerstoff (O) oder Schwefel (S) bedeutet, bevorzugt Sauerstoff,
- R⁵, R⁶, R⁷ und R⁸: gleich oder verschieden sind und Wasserstoff, organische oder anorganische Reste darstellen, bevorzugt Wasserstoff oder -SO₂-N(CH₃)₂, oder-NH-CO-CF₃,
- R⁹: einen Alkyl-, Cycloalkyl-, Alkenyl-, Alkinyl-, Aryl-, Alkoxy-, Alkenyloxy-, Alkinyloxy-, Aryloxy-, Alkoxycarbonyl-, Alkylamino-, Alkylthio-, Arylthio-, Alkylsulfonyl- oder Alkylsulfinyl-Rest darstellt, bevorzugt i-Propyl, die alle jeweils optional durch ein oder mehrere Alkyl-, Halogen-, Alkoxy-, Aryl- oder Heteroaryl-Reste substituiert sein können, und
- L: ein Ligand ist, der die gleichen Bedeutungen besitzt wie für die Formel (A) genannt.

Die Katalysatoren der Formel (B) sind prinzipiell bekannt. Vertreter dieser Verbindungsklasse sind die Katalysatoren, die von Hoveyda et al. in US 2002/0107138 A1 und Angew. Chem. Int. Ed. 2003, 42, 4592 beschrieben sind, und die Katalysatoren, die von Grela in WO-A-2004/035596, Eur. J. Org. Chem 2003, 963-966 und Angew. Chem. Int. Ed. 2002, 41, 4038 sowie in J. Org. Chem. 2004, 69, 6894-96 und Chem. Eur. J 2004, 10, 777-784 beschrieben werden. Die Katalysatoren der Formel (B) sind käuflich erhältlich bzw. gemäß den angegebenen Literaturstellen herstellbar.
Als besonders bevorzugter Ruthenium Übergangsmetallkatalysator wird wenigstens einer aus der Gruppe
**M1** = Benzyliden[1,3-bis(2,4,6-trimethylphenyl)-2-imidazolidinyliden]dichloro(tricyclohexyl-phosphin)ruthenium (Grubbs II Katalysator, CAS No. 246047-72-3),
**M2** = Bis(tricyclohexylphosphine)-[(phenylthio)methylene]ruthenium(II)dichloride (CAS No. 219770-99-7),
**M3** = 1,3-Bis(2,4,6-trimethylphenyl)-4,5-dihydroimidazol-2-ylidene[2-(i-propoxy)-5-(N,N-di-MeNH₂SO₂)phenyl]methyleneruthenium(II)dichloride (Zhan Kat. 1B, CAS No. 918870-76-5),
**M4** = Bis(tricyclohexylphosphine)-3-phenyl-1H-inden-1-ylideneruthenium(II)dichloride (CAS No. 250220-36-1),
**M5** = Benzylidenbis(tricyclohexylphosphin)dichlororuthenium (Grubbs I Katalysator, CAS No. 172222-30-9),
**M6** = Dichloro(o-isopropoxyphenylmethylene)(tricyclohexylphosphine)ruthenium(II) (Hoveyda-Grubbs Kat.I, CAS No. 203714-71-0),
**M7** = (1,3-Bis-(2,4,6-trimethylphenyl)-2-imidazolidinylidene)dichloro(o-isopropoxyphenyl methylene)ruthenium (Hoveyda-Grubbs Kat.II, CAS No.301224-40-8),
**M8** = Tricyclohexylphosphine[4,5-dimethyl-1,3-bis(2,4,6-trimethylphenyl)imidazol-2-ylidene][2-thienylmethylene]ruthenium(II)dichloride, min. 95% (CAS No. 1190427-50-9),
**M9** = Tricyclohexylphosphine[1,3-bis(2,4,6-trimethylphenyl)imidazol-2-ylidene][3-phenyl-1H-inden-1-ylidene]ruthenium(II)dichloride, min. 95% (CAS No. 254972-49-1),
**M10** = [1,3-Bis(2,6-di-i-propylphenyl)-4,5-dihydroimidazol-2-ylidene]-[2-i-propoxy-5-(trifluoroacetamido)phenyl]methyleneruthenium(II)dichloride (CAS No. 1212008-99-5),
**M11** = Tri(i-propoxy)phosphine(3-phenyl-1H-inden-1-ylidene)[1,3-bis(2,4,6-trimethyl phenyl)-4,5-dihydroimidazol-2-ylidene]ruthenium(II)dichloride, min. 95% (Strem Chemicals Katalog Nummer 44-7783),
**M12** = Tricyclohexylphosphine[1,3-bis(2,4,6-trimethylphenyl)imidazol-2-ylidene][2-thienyl methylene]ruthenium(II)dichloride, min. 95% (CAS No. 1190427-49-6),
**M13** = 3-Phenyl-1H-inden-1-ylidene[bis(i-butylphoban)]ruthenium(II)dichloride (CAS No. 894423-99-5),
**M14 =** Dichloro[1,3-bis(2-methylphenyl)-2-imidazolindinyliden](benzylidene) (tricyclohexyl phosphine)ruthenium(II) (CAS No. 927429-60-5),
**M15** = Dichloro[1,3-bis(2-methylphenyl)-2-imidazolindinyliden](2-isopropoxy-phenyl methylene)ruthenium(II) (CAS No. 927429-61-6)

In der erfindungsgemäßen 1,3-Butadien Synthese eingesetzt. Insbesondere bevorzugt wird als Übergangsmetallkatalysator des Elementes Ruthenium **M5** eingesetzt.

Die Herstellung der erfindungsgemäß einzusetzenden Katalysatoren ist dem Fachmann bekannt. Beispiele finden sich in P. Schwab, R. H. Grubbs, J. W. Ziller, J. Am. Chem. Soc., 1996, 118 (1), S. 100-110. Die Katalysatoren **M1** bis **M15** sind zudem käuflich zu erwerben bei Sigma-Aldrich Laborchemikalien GmbH, Seelze, Deutschland oder Strem Chemicals Inc. Kehl, Deutschland.

Die Menge der erfindungsgemäß einzusetzenden Übergangsmetallkatalysatoren des Elementes Ruthenium im Reaktionsgemisch liegt üblicherweise im Bereich von 0,01 bis 10000 Mol.-%, bevorzugt 0,1 bis 100 Mol.-%, besonders bevorzugt 0,2 bis 10 Mol.-% gerechnet als Summe aller einzusetzenden Katalysatoren und bezogen auf die eingesetzte Menge Ethen.

Bevorzugt liegt der Gesamtgasfluss, also das als Edukt der Reaktionsapparatur zuzuführende Gemisch aus Ethen und Ethin, in einem Bereich von 200 bis 500 l/h, besonders bevorzugt im Bereich von 200 bis 400 l/h, ganz besonders bevorzugt im Bereich von 200 bis 300 l/h bei einem Lösungsmittelvolumen von 1 I. Der Fachmann kann auf Basis dieser Angaben die entsprechenden Parameter für den Ansatz kleinerer oder größerer Ansätze errechnen.

Im Gesamtgasfluss liegen die Edukte Ethen und Ethin bevorzugt im Verhältnis im Bereich von 6:4 bis 4:6 vor, insbesondere bevorzugt im Verhältnis im Bereich von 1:1 vor.

In bevorzugter Ausführungsform wird der Gasstrom enthaltend das Gemisch aus Ethen und Ethin vor dem Eintritt in den Reaktionsapparat getrocknet. Dem Fachmann sind geeignete Trockenmittel bekannt. Bevorzugt wird wenigstens ein Trockenmittel aus der Gruppe konzentrierte Schwefelsäure, Natronlauge und Molsieb zur Trocknung des erfindungsgemäß einzusetzenden Gasgemisches eingesetzt. Insbesondere bevorzugt werden alle drei Trocknungsmittel nacheinander eingesetzt.

Für die erfindungsgemäße 1,3-Butadien Synthese eignet sich jede Art von Reaktionsgefäß bzw. Apparatur, das bzw. die üblicherweise von einem Fachmann zum Umsetzen von Gasen eingesetzt wird. Bevorzugt sind Rührkesselreaktoren, Strömungsrohre oder Blasensäulenreaktoren. Vorzugsweise sind diese aus Stahl hergestellt. Für kleinere Ansätze können auch Glas basierte Reaktoren eingesetzt werden. Die Begriffe Reaktionsgefäß, Apparatur und Reaktor werden in der vorliegenden Anmeldung synonym verwendet.

Im Allgemeinen geht man bei der Reaktionsführung so vor, dass der wenigstens eine Übergangsmetallkatalysator des Elementes Ruthenium, im Falle eines Katalysatorgemisches gegebenenfalls vorgemischt, als solcher oder vorzugsweise gelöst in wenigstens einem der oben genannten Lösungsmittel, gegebenenfalls unter Kühlung aber unter Schutzgas, insbesondere Stickstoff, in wenigstens ein gekühltes Reaktionsgefäß gegeben wird.

Bevorzugt wird das wenigstens eine Reaktionsgefäß auf Temperaturen im Bereich von - 70 bis 50 °C, besonders bevorzugt auf Temperaturen im Bereich von 15 bis 40 °C temperiert. Durch einen Einlass wird ein Gemisch aus gereinigtem Ethin und Ethen eingeleitet, bevorzugt im Falle des Einsatzes von Lösungsmittel unterhalb des Flüssigkeitsspiegels.

Nicht umgesetzter Anteil des Gasgemisches kann entweder dem Reaktionsgefäß wieder zugeführt werden, oder in alternativer Ausführungsform, in wenigstens ein zweites Reaktionsgefäß überführt werden, das ebenfalls wenigstens einen erfindungsgemäß einzusetzenden Übergangsmetallkatalysator des Elementes Ruthenium enthält. Auch hier erfolgt die Einleitung des Eduktgemisches aus Ethen und Ethin im Falle des Einsatzes von Lösungsmittel unterhalb des Flüssigkeitsspiegels. Hieraus entweichendes nicht umgesetztes Gasgemisch kann entweder wiederum dem ersten Reaktionsgefäß zugeführt werden, oder dem wenigstens einen zweiten Reaktionsgefäß erneut zugeführt werden, oder an die Umgebungsluft / Abluft abgegeben werden.

Die Handhabung des Ethins ist insbesondere unter Überdruck sicherheitstechnisch anspruchsvoll, weshalb das die Edukte enthaltende Gasgemisch bevorzugt nur bei schwachem Überdruck von bis zu 100 mbarü durch das katalysatorhaltige Lösungsmittel geleitet wird.

Mit einem Siedepunkt von - 4,5 °C reichert sich das 1,3-Butadien im Lösungsmittel an und kann von diesem sowie anfallenden Nebenprodukten destillativ getrennt werden. Der Abbruch der erfindungsgemäßen Synthese von 1,3-Butadien aus Ethen und Ethin und die Aufreinigung der Reaktionsprodukte können nach allgemein dem Fachmann bekannten Verfahren vorgenommen werden. Das Lösungsmittel kann auch dazu verwendet werden, nach Abtrennung der Reaktionsprodukte den wenigstens einen Katalysator der Reaktion wieder zur Verfügung zu stellen.

### Beispiele

### Einsatzstoffe

Ethen, CAS Nr. 74-85-1, Reinheit 99,9 %, bezogen von Linde AG
Ethin, CAS Nr. 74-86-2, Reinheit 99,5 %, bezogen von Linde AG
Katalysatoren: **M1** bis **M15** gemäß Beschreibung von Sigma-Aldrich Laborchemikalien GmbH, Seelze, Deutschland oder Strem Chemicals Inc. Kehl, Deutschland
Schutzgas: Stickstoff

### Apparativer Aufbau

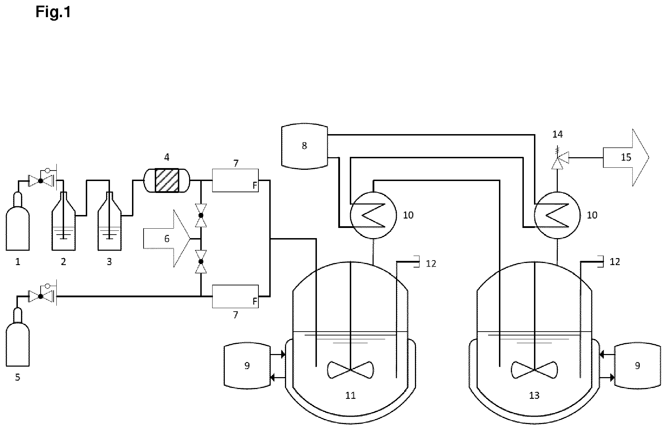

**Fig. 1** zeigt beispielhaft einen apparativen Aufbau, wie er für die Versuche im Rahmen der vorliegenden Erfindung verwendet wurde. In **Fig. 1** bedeuten
1 Ethin
2 H₂SO₄ (95 % - 98 %)
3 2 N NaOH
4 Molsieb 4 Ä (Sigma Aldrich)
5 Ethen
6 Schutzgas N₂
7 Blasenzähler
8 Kryostat -20°C
9 Heizbad
10 Intensivkühler
11 Reaktor 1, 250 ml, Glas
12 Septum
13 Reaktor 2, 250 ml, Glas
14 circa 100 mbarü
15 Abluft

Die Versuchsapparatur bestand aus zwei Reaktoren (250-ml-Dreihals-Glaskolben) mit Rührfisch, zwei Intensivkühlern (Kühlflüssigkeit temperiert auf - 20 °C), zwei Septi zur Probenahme und zwei Ölbadern als Heizbad. Über eine Glas-Kapillare mit einem Durchmesser zwischen 1,5 und 7 mm wurde das Gasgemisch aus zuvor gereinigtem Ethin (zuerst 95 - 98%ige H₂SO₄ danach 2 N NaOH sowie schließlich Molsieb) und Ethen zunächst in einen ersten Reaktor unterhalb des Flüssigkeitsspiegels eingeleitet. Derjenige Anteil dieses Gasgemisches, welcher nicht durch Reaktion verbraucht oder im Intensivkühler des Reaktor 1 kondensierte, wurde wiederum über eine weitere Kapillare unterhalb des Flüssigkeitsspiegels in einen zweiten Reaktor geleitet. Nach Durchgang durch den zweiten Intensivkühler gelangten die nicht umgesetzten oder nicht im Intensivkühler des Reaktor 2 kondensierten Anteile des Gasgemisches in die Abluft.

Der flüssige Reaktorinhalt aus Reaktor 1 und Reaktor 2 wurde mittels GC (Gaschromatographie) untersucht. Aufgrund der geringen Umsätze erfolgte die Auswertung nur qualitativ. Die Anwesenheit der Referenzsubstanz Cyclohexan erlaubte jedoch, die gebildete Butadienmenge über das Peakflächenverhältnis Butadien zu Cyclohexan für die Versuche im Rahmen der vorliegenden Erfindung zu vergleichen.

**Tabelle 1** zeigt die erfassten Substanzen mit ihren jeweiligen Retentionszeiten. Die Identifikation erfolgte durch Vergleich mit kommerziellen Reinsubstanzen oder mittels GC-MS (MS = Massenspektrometrie).

**Tabelle 1**

| **Retentionszeit /min** | **Substanz** |
|---|---|
| **2,08** | Ethen, Ethin, Luft |
| **2,26** | 1,3-Butadien |
| **2,69** | *trans*-1,3-Pentadien |
| **2,75** | *cis*-1,3-Pentadien |
| **2,88** | Dichlormethan |
| **3,64** | Cyclohexan |
| **4,14; 4,35; 4,37** | 2,4-Hexadien-Isomere |
| **4,70** | Benzol |
| **7,01** | Toluol |
| **8,94** | Octatetraen |
| **9,25** | Styrol |

### Vergleichsbeispiel 1 (ohne Katalysator)

50 ml Dichlormethan wurden unter Schutzgas in einem zuvor mittels N₂ inertisierten 100 ml Glasgefäß mit Intensivkühler (Temperatur des Kühlmediums -20 °C) auf 30 °C temperiert. Die Inertisierung von Räumen bezeichnet den Vorgang, durch Zugabe von inerten Gasen oder Dämpfen den Luftsauerstoff oder reaktions- bzw. explosionsfähige Gase oder Gasgemische aus Räumen zu verdrängen. Zum Dichlormethan wurden außerdem 1,0 ml Cyclohexan als Referenz zugegeben. Anschließend wurde unter Rühren während der gesamten Reaktionszeit ein Gemisch aus Ethen und Ethin im Verhältnis 1:1 ins Lösungsmittel eingeleitet, wobei in regelmäßigen Abständen Proben aus der Flüssigphase entnommen wurden. Es wurde kein Butadien gebildet.

### Vergleichsbeispiel 2 (M4, ohne Ethen)

50 mg Katalysator **M4,** gelöst in 50 ml Dichlormethan, wurden unter Schutzgas in einem zuvor mittels N₂ inertisierten Gefäß mit Intensivkühler (Temperatur des Kühlmediums -20 °C) auf 30 °C temperiert. Außerdem wurden 1,0 ml Cyclohexan als Referenz zugegeben. Anschließend wurde unter Rühren während der gesamten Reaktionszeit Ethin eingeleitet und in regelmäßigen Abständen Proben aus der Flüssigphase entnommen. Es wurde kein Butadien gebildet.

### Vergleichsbeispiel 3 (M5, ohne Ethen)

50 mg Katalysator **M5,** gelöst in 50 ml Dichlormethan, wurden unter Schutzgas in einem zuvor inertisierten Gefäß mit Intensivkühler (Temperatur des Kühlmediums -20 °C) auf 30 °C temperiert. Außerdem wurden 1,0 ml Cyclohexan als Referenz zugegeben. Anschließend wurde unter Rühren während der gesamten Reaktionszeit Ethin eingeleitet und in regelmäßigen Abständen Proben aus der Flüssigphase entnommen. Es wurde kein Butadien gebildet.

### Beispiel 1

50 mg Katalysator **MX,** wobei **MX** für jeweils einen der oben genannten Katalysatoren **M1** bis **M15** steht, gelöst in 50 ml Dichlormethan, wurden unter Schutzgas (N₂) in einem zuvor mit N₂ inertisierten Gefäß mit Intensivkühler (Temperatur des Kühlmediums -20 °C) auf 30 °C temperiert. Außerdem wurden 1,0 ml Cyclohexan als Referenz zugegeben. Anschließend wurde unter Rühren während der gesamten Reaktionszeit ein Gemisch aus Ethen und Ethin im Verhältnis 1:1 unterhalb des Flüssigkeitsspiegels ins Lösungsmittel eingeleitet. Während der Reaktionszeit wurden in regelmäßigen Abständen Proben aus der Flüssigphase entnommen. Per Gaschromatographie wurde in diesen Proben Butadien nachgewiesen.

### Beispiel 2 (M5, -64 °C)

50 mg Katalysator **M5,** gelöst in 50 ml Dichlormethan, wurden unter Schutzgas in einem zuvor mittels N₂ inertisierten Gefäß mit Intensivkühler (Temperatur des Kühlmediums -20 °C) auf -64 °C temperiert. Außerdem wurden 1,0 ml Cyclohexan als Referenz zugegeben. Anschließend wurde unter Rühren während der gesamten Reaktionszeit ein Gemisch aus Ethen und Ethin im Verhältnis 1:1 eingeleitet. Während der Reaktionszeit wurden in regelmäßigen Abständen Proben aus der Flüssigphase entnommen. Per Gaschromatographie wurde in diesen Proben Butadien nachgewiesen.

### Beispiel 3 (M5, doppelte Katalysatormenge)

100 mg Katalysator **M5,** gelöst in 50 ml Dichlormethan, wurden unter Schutzgas (N₂) in einem zuvor mittels N₂ inertisierten Gefäß mit Intensivkühler (Temperatur des Kühlmediums -20 °C) auf 30 °C temperiert. Außerdem wurden 1,0 ml Cyclohexan als Referenz zugegeben. Anschließend wurde unter Rühren während der gesamten Reaktionszeit ein Gemisch aus Ethen und Ethin im Verhältnis 1:1 eingeleitet. Während der Reaktionszeit wurden in regelmäßigen Abständen Proben aus der Flüssigphase entnommen. Per Gaschromatographie wurde in diesen Proben Butadien nachgewiesen.

Die Vergleichsbeispiele 1 bis 3 zeigen, dass zwei Komponenten essentiell sind für die Durchführung des erfindungsgemäßen Verfahrens: Ethin muss erstens mit Katalysator und zweitens mit Ethen kontaktiert werden.

## Patentansprüche

1. Verfahren zur Herstellung von 1,3-Butadien, **dadurch gekennzeichnet, dass** man Ethen und Ethin in Gegenwart mindestens eines homogenen Übergangsmetallkatalysators des Elementes Ruthenium miteinander umsetzt.

2. Verfahren gemäß Anspruch 1, **dadurch gekennzeichnet, dass** dieses in flüssiger Phase mit wenigstens einem Lösungsmittel durchgeführt wird.

3. Verfahren gemäß Anspruch 2, **dadurch gekennzeichnet, dass** als Lösungsmittel ein bei Raumtemperatur flüssiges Halogenalkan, bevorzugt Dichlormethan, eingesetzt wird.

4. Verfahren gemäß einem der Ansprüche 1 bis 3, **dadurch gekennzeichnet, dass** dieses bei Temperaturen im Bereich von -70 bis 50 °C durchgeführt wird.

5. Verfahren gemäß einem der Ansprüche 1 bis 4, **dadurch gekennzeichnet, dass** dieses ohne Überdruck durchgeführt wird.

6. Verfahren gemäß einem der Ansprüche 1 bis 5, **dadurch gekennzeichnet dass** mindestens ein Übergangsmetallkatalysator der allgemeinen Formel (A) eingesetzt wird, 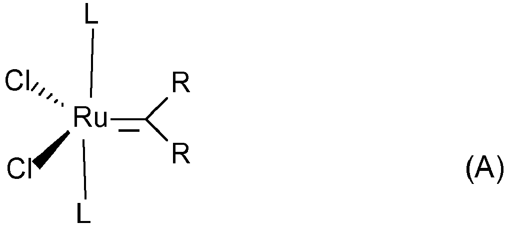 worin
L gleiche oder verschiedene Liganden, bevorzugt neutrale Elektronen-Donoren darstellen, und
R gleich oder verschieden sind und Wasserstoff, Alkyl, eine nicht-aromatische carbocyclische Gruppe, die cyclisierte Alkyl, Alkenyl oder Alkinyl-Gruppen enthält, wie etwa Cycloalkyl, Alkenyl, Alkinyl, Aryl, Carboxylat, Alkoxy, Alkenyloxy, Alkinyloxy, Aryloxy, Alkoxycarbonyl, Alkylamino, Alkylthio, Arylthio, Alkylsulfonyl oder Alkylsulfinyl bedeuten, wobei diese Reste alle jeweils optional durch ein oder mehrere Alkyl-, Halogen-, Alkoxy-, Aryl- oder Heteroaryl-Reste substituiert sein können, oder alternativ beide Reste R unter Einbindung des gemeinsamen C-Atoms, an das sie gebunden sind, zu einer cyclischen Gruppe verbrückt sind, die aliphatischer oder aromatischer Natur sein kann, gegebenenfalls substituiert ist und ein oder mehrere Heteroatome enthalten kann.

7. Verfahren gemäß Anspruch 6, **dadurch gekennzeichnet dass** R in der allgemeinen Formel (A) jeweils unabhängig voneinander für Wasserstoff, C₁-C₃₀-Alkyl, eine nicht-aromatische carbocyclische C₃-C₂₀-Gruppe, die cyclisierte Alkyl, Alkenyl oder Alkinyl-Gruppen enthält, wie etwa C₃-C₂₀-Cycloalkyl, C₂-C₂₀-Alkenyl, C₂-C₂₀-Alkinyl, C₆-C₂₄-Aryl, C₁-C₂₀-Carboxylat, C₁-C₂₀-Alkoxy, C₂-C₂₀-Alkenyloxy, C₂-C₂₀-Alkinyloxy, C₆-C₂₄-Aryloxy, C₂-C₂₀-Alkoxycarbonyl, C₁-C₃₀-Alkylamino, C₁-C₃₀-Alkylthio, C₆-C₂₄-Arylthio, C₁-C₂₀-Alkylsulfonyl oder C₁-C₂₀-Alkylsulfinyl, wobei diese Reste alle jeweils optional durch ein oder mehrere C₁-C₆-Alkyl-, Halogen-, C₁-C₆-Alkoxy-, bevorzugt i-Propoxy-, Aryl-, bevorzugt Phenyl, oder Heteroaryl-Reste substituiert sein können, oder alternativ beide Reste R unter Einbindung des gemeinsamen C-Atoms, an das sie gebunden sind, zu einer cyclischen Gruppe verbrückt sind, die aliphatischer oder aromatischer Natur sein kann, gegebenenfalls substituiert ist und ein oder mehrere Heteroatome enthalten kann.

8. Verfahren gemäß einem der Ansprüche 1 bis 5, **dadurch gekennzeichnet, dass** als Übergangsmetallkatalysator des Elementes Ruthenium wenigstens einer aus der Gruppe
**M1 =** Benzyliden[1,3-bis(2,4,6-trimethylphenyl)-2-imidazolidinyliden]dichloro (tricyclohexyl-phosphin)ruthenium,
**M2 =** Bis(tricyclohexylphosphine)-[(phenylthio)methylene]ruthenium(II)dichloride,
**M3 =** 1,3-Bis(2,4,6-trimethylphenyl)-4,5-dihydroimidazol-2-ylidene[2-(i-propoxy)-5-(N,N-di-MeNH₂SO₂)phenyl]methyleneruthenium(II)dichloride,
**M4 =** Bis(tricyclohexylphosphine)-3-phenyl-1H-inden-1-ylideneruthenium(II)di chloride,
**M5 =** Benzylidenbis(tricyclohexylphosphin)dichlororuthenium,
**M6 =** Dichloro(o-isopropoxyphenylmethylene)(tricyclohexylphosphine) ruthenium(II),
**M7 =** (1,3-Bis-(2,4,6-trimethylphenyl)-2-imidazolidinylidene)dichloro(o-isopropoxyphenyl methylene)ruthenium,
**M8 =** Tricyclohexylphosphine[4,5-dimethyl-1,3-bis(2,4,6-trimethylphenyl)imidazol-2-ylidene][2-thienylmethylene]ruthenium(II)dichloride,
**M9 =** Tricyclohexylphosphine[1,3-bis(2,4,6-trimethylphenyl)imidazol-2-ylidene][3-phenyl-1H-inden-1-ylidene]ruthenium(II)dichloride,
**M10 =** [1,3-Bis(2,6-di-i-propylphenyl)-4,5-dihydroimidazol-2-ylidene]-[2-i-propoxy-5-(trifluoroacetamido)phenyl]methyleneruthenium(II)dichloride,
**M11 =** Tri(i-propoxy)phosphine(3-phenyl-1H-inden-1-ylidene)[1,3-bis(2,4,6-trimethyl phenyl)-4,5-dihydroimidazol-2-ylidene]ruthenium(II)dichloride,
**M12 =** Tricyclohexylphosphine[1,3-bis(2,4,6-trimethylphenyl)imidazol-2-ylidene][2-thienyl methylene]ruthenium(II)dichloride,
**M13 =** 3-Phenyl-1H-inden-1-ylidene[bis(i-butylphoban)]ruthenium(II)dichloride,
**M14 =** Dichloro[1,3-bis(2-methylphenyl)-2-imidazolindinyliden](benzylidene) (tricyclohexyl phosphine)ruthenium(II),
**M15 =** Dichloro[1,3-bis(2-methylphenyl)-2-imidazolindinyliden](2-isopropoxy-phenyl methylene)ruthenium(II),
eingesetzt wird.

9. Verfahren gemäß Anspruch 6, **dadurch gekennzeichnet, dass** als Übergangsmetallkatalysator des Elementes Ruthenium wenigstens **M5** eingesetzt wird.

10. Verfahren gemäß einem der Ansprüche 1 bis 7, **dadurch gekennzeichnet, dass** die Menge des wenigstens einen Katalysators im Reaktionsgemisch im Bereich von 0,01 bis 10000 Mol.-% gerechnet als Summe aller eingesetzten Katalysatoren und bezogen auf die eingesetzte Menge Ethen liegt.

11. Verfahren gemäß einem der Ansprüche 1 bis 8, **dadurch gekennzeichnet, dass** man dieses absatzweise durchführt.

12. Verwendung von wenigstens einem homogenen Übergangsmetallkatalysator des Elementes Ruthenium zur Herstellung von 1,3-Butadien aus Ethen und Ethin.

## Claims

1. Process for preparing 1,3-butadiene, **characterized in that** ethene and ethyne are reacted with one another in the presence of at least one homogeneous transition metal catalyst of the element ruthenium.

2. Process according to Claim 1, **characterized in that** it is carried out in liquid phase using at least one solvent.

3. Process according to Claim 2, **characterized in that** a haloalkane which is liquid at room temperature, preferably dichloromethane, is used as solvent.

4. Process according to any of Claims 1 to 3, **characterized in that** it is carried out at temperatures in the range from -70 to 50°C.

5. Process according to any of Claims 1 to 4, **characterized in that** it is carried out without superatmospheric pressure.

6. Process according to any of Claims 1 to 5, **characterized in that** at least one transition metal catalyst of the general formula (A) is used, 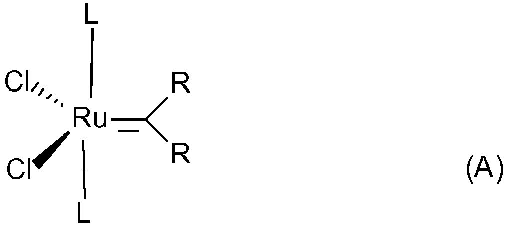 wherein
the radicals L are identical or different ligands, preferably uncharged electron donors, the radicals R are identical or different and are each hydrogen, alkyl, a nonaromatic carbocyclic group which contains cyclized alkyl, alkenyl or alkynyl groups such as cycloalkyl, alkenyl, alkynyl, aryl, carboxylate, alkoxy, alkenyloxy, alkynyloxy, aryloxy, alkoxycarbonyl, alkylamino, alkylthio, arylthio, alkylsulphonyl or alkylsulphinyl, where these radicals can each optionally be substituted by one or more alkyl, halogen, alkoxy, aryl or heteroaryl radicals, or, as an alternative, the two radicals R are bridged with inclusion of the common carbon atom to which they are bound to form a cyclic group which can be aliphatic or aromatic in nature and is optionally substituted and can contain one or more heteroatoms.

7. Process according to Claim 6, **characterized in that** R in the general formula (A) is in each case, independently of one another, hydrogen, C₁-C₃₀-alkyl, a nonaromatic carbocyclic C₃-C₂₀ group which contains cyclized alkyl, alkenyl or alkynyl groups such as C₃-C₂₀-cycloalkyl, C₂-C₂₀-alkenyl, C₂-C₂₀-alkynyl, C₆-C₂₄-aryl, C₁-C₂₀-carboxylate, C₁-C₂₀-alkoxy, C₂-C₂₀-alkenyloxy, C₂-C₂₀-alkynyloxy, C₆-C₂₄-aryloxy, C₂-C₂₀-alkoxycarbonyl, C₁-C₃₀-alkylamino, C₁-C₃₀-alkylthio, C₆-C₂₄-arylthio, C₁-C₂₀-alkylsulphonyl or C₁-C₂₀-alkylsulphinyl, where these radicals can in each case optionally be substituted by one or more C₁-C₆-alkyl, halogen, C₁-C₆-alkoxy, preferably i-propoxy, aryl, preferably phenyl, or heteroaryl radicals, or, as an alternative, the two radicals R are bridged with inclusion of the common carbon atom to which they are bound to form a cyclic group which can be aliphatic or aromatic in nature and is optionally substituted and can contain one or more heteroatoms.

8. Process according to any of Claims 1 to 5, **characterized in that** at least one catalyst from the group
**M1** = benzylidene[1,3-bis(2,4,6-trimethylphenyl)-2-imidazolidinylidene]dichloro(tricyclohexylpho sphine)ruthenium,
**M2**=bis(tricyclohexylphosphine)[(phenylthio)methyle ne]ruthenium(II) dichloride,
**M3** = 1,3-bis(2,4,6-trimethylphenyl)-4,5-dihydroimidazol-2-ylidene[2-(i-propoxy)-5-(N,N-di-MeNH₂SO₂)phenyl]methyleneruthenium(II) dichloride,
**M4** = bis(tricyclohexylphosphine)-3-phenyl-1H-inden-1-ylideneruthenium(II) dichloride,
**M5**=benzylidenebis(tricyclohexylphosphine)dichloror uthenium,
**M6** = dichloro(o-isopropoxyphenylmethylene)-(tricyclohexylphosphine)ruthenium(II),
**M7** = (1,3-bis(2,4,6-trimethylphenyl)-2-imidazolidinylidene)dichloro(o-isopropoxyphenyl methylene)ruthenium,
**M8** = tricyclohexylphosphine[4,5-dimethyl-1,3-bis(2,4,6-trimethylphenyl)imidazol-2-ylidene][2-thienylmethylene]ruthenium(II) dichloride,
**M9** = tricyclohexylphosphine[1,3-bis(2,4,6-trimethylphenyl)imidazol-2-ylidene][3-phenyl-1H-inden-1-ylidene]ruthenium(II) dichloride,
**M10=** [1,3-bis(2,6-di-i-propylphenyl)-4,5-dihydroimidazol-2-ylidene]-[2-i-propoxy-5-(trifluoroacetamido)phenyl]methyleneruthenium (II) dichloride,
**M11**= tri(i-propoxy)phosphine(3-phenyl-1H-inden-1-ylidene)[1,3-bis(2,4,6-trimethyl phenyl)-4,5-dihydroimidazol-2-ylidene]ruthenium(II) dichloride,
**M12=** tricyclohexylphosphine[1,3-bis(2,4,6-trimethylphenyl)imidazol-2-ylidene][2-thienyl methylene]ruthenium(II) dichloride,
**M13=** 3-phenyl-1H-inden-1-ylidene[bis(i-butylphobane)]ruthenium(II) dichloride,
**M14=** dichloro[1,3-bis(2-methylphenyl)-2-imidazolindinylidene] (benzylidene) (tricyclohexyl phosphine)ruthenium(II),
**M15=** dichloro[1,3-bis(2-methylphenyl)-2-imidazolindinylidene](2-isopropoxyphenyl methylene)ruthenium(II),

9. Process according to Claim 6, **characterized in that** at least **M5** is used as transition metal catalyst of the element ruthenium.

10. Process according to any of Claims 1 to 7, **characterized in that** the amount of the at least one catalyst in the reaction mixture is in the range from 0.01 to 10 000 mol%, calculated as sum of all catalysts used and based on the amount of ethene used.

11. Process according to any of Claims 1 to 8, **characterized in that** it is carried out batchwise.

12. Use of at least one homogeneous transition metal catalyst of the element ruthenium for preparing 1,3-butadiene from ethene and ethyne.

## Revendications

1. Procédé pour la préparation 1,3-butadiène, **caractérisé en ce qu'**on fait réagir l'un avec l'autre de l'éthène et de l'éthyne en présence d'au moins un catalyseur homogène à base d'un métal de transition de l'élément ruthénium.

2. Procédé selon la revendication 1, **caractérisé en ce qu'**il est réalisé en phase liquide contenant au moins un solvant.

3. Procédé selon la revendication 2, **caractérisé en ce qu'**un halogénoalcane liquide à température ambiante, de préférence du dichlorométhane, est utilisé comme solvant.

4. Procédé selon l'une quelconque des revendications 1 à 3, **caractérisé en ce qu'**il est réalisé à des températures dans la plage de -70 à 50°C.

5. Procédé selon l'une quelconque des revendications 1 à 4, **caractérisé en ce qu'**il est réalisé sans surpression.

6. Procédé selon l'une quelconque des revendications 1 à 5, **caractérisé en ce qu'**au moins un catalyseur à base de métal de transition de formule générale (A) est utilisé, 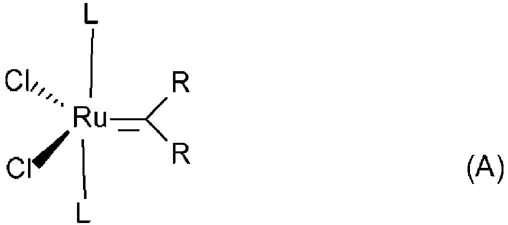 dans laquelle
L représentent des ligands identiques ou différents, de préférence des donneurs d'électrons neutres, et
R sont identiques ou différents et signifient hydrogène, alkyle, un groupe carbocyclique non aromatique, qui contient des groupes alkyle, alcényle ou alcényle cyclisés, tels que par exemple cycloalkyle, alcényle, alcynyle, aryle, carboxylate, alcoxy, alcényloxy, alcynyloxy, aryloxy, alcoxycarbonyle, alkylamino, alkylthio, arylthio, alkylsulfonyle ou alkylsulfinyle, ces radicaux pouvant tous à chaque fois éventuellement être substitués par un ou plusieurs radicaux alkyle, halogène, alcoxy, aryle ou hétéroaryle, ou, en variante, les deux radicaux R sont pontés, avec incorporation de l'atome de carbone commun auquel ils sont liés, en un groupe cyclique, qui peut être de nature aliphatique ou aromatique, qui est le cas échéant substitué et qui peut contenir un ou plusieurs hétéroatomes.

7. Procédé selon la revendication 6, **caractérisé en ce que** les radicaux R dans la formule générale (A) signifient à chaque fois, indépendamment l'un de l'autre, hydrogène, C₁-C₃₀-alkyle, un groupe C₃-C₂₀ carbocyclique non aromatique, qui contient des groupes alkyle, alcényle ou alcynyle cyclisés, tels que par exemple C₃-C₂₀-cycloalkyle, C₂-C₂₀-alcényle, C₂-C₂₀-alcynyle, C₆-C₂₄-aryle, C₁-C₂₀-carboxylate, C₁-C₂₀-alcoxy, C₂-C₂₀-alcényloxy, C₂-C₂₀-alcynyloxy, C₆-C₂₄-aryloxy, C₂-C₂₀-alcoxycarbonyle, C₁-C₃₀-alkylamino, C₁-C₃₀-alkylthio, C₆-C₂₄-arylthio, C₁-C₂₀-alkylsulfonyle ou C₁-C₂₀-alkylsulfinyle, ces radicaux pouvant tous à chaque fois éventuellement être substitués par un ou plusieurs radicaux C₁-C₆-alkyle, halogène, C₁-C₆-alcoxy, de préférence i-propoxy, aryle, de préférence phényle, ou hétéroaryle ou, en variante, les deux radicaux R sont pontés, avec incorporation de l'atome de carbone commun auquel ils sont liés, en un groupe cyclique, qui peut être de nature aliphatique ou aromatique, qui est le cas échéant substitué et qui peut contenir un ou plusieurs hétéroatomes.

8. Procédé selon l'une quelconque des revendications 1 à 5, **caractérisé en ce qu'**on utilise, comme catalyseur à base de métal de transition de l'élément ruthénium, au moins l'un du groupe formé par
M1 = benzylidène[1,3-bis(2,4,6-triméthylphényl)-2-imidazolidinylidène]dichloro-(tricyclohexylphosphine)-ruthénium,
M2 = dichlorure de bis(tricyclohexylphosphine)-[(phénylthio)méthylène]ruthénium (II),
M3 = dichlorure de 1,3-bis(2,4,6-triméthylphényl)-4,5-dihydroimidazol-2-ylidène[2-(i-propoxy)-5-(N,N-di-MeNH₂SO₂)phényl]méthylèneruthénium (II),
M4 = dichlorure de bis(tricyclohexylphosphine)-3-phényl-1H-indène-1-ylidèneruthénium (II),
M5 = benzylidène-bis(tricyclohexylphosphine)-dichlororuthénium,
M6 = dichloro(o-isopropoxyphénylméthylène)-(tricyclohexylphosphine)-ruthénium (II),
M7 = (1,3-bis-(2,4,6-triméthylphényl)-2-imidazolidinylidène)dichloro(o-isopropoxyphényl-méthylène)-ruthénium,
M8 = dichlorure de tricyclohexylphosphine[4,5-diméthyl-1,3-bis(2,4,6-triméthylphényl)imidazol-2-ylidène][2-thiénylméthylène]ruthénium (II),
M9 = dichlorure de tricyclohexylphosphine[1,3-bis(2,4,6-triméthylphényl)imidazol-2-ylidène] [3-phényl-1H-indène-1-ylidène]ruthénium (II),
M10 = dichlorure de [1,3-bis(2,6-di-i-propylphényl)-4,5-dihydro-imidazol-2-ylidène]-[2-i-propoxy-5-(trifluoroacétamido)phényl]méthylèneruthénium (II),
M11 = dichlorure de tri(i-propoxy)phosphine(3-phényl-1H-indène-1-ylidène)[1,3-bis(2,4,6-triméthylphényl)-4,5-dihydroimidazol-2-ylidène]ruthénium (II),
M12 = dichlorure de tricyclohexylphosphine[1,3-bis(2,4,6-triméthylphényl)imidazol-2-ylidène][2-thiénylméthylène]ruthénium (II),
M13 = dichlorure de 3-phényl-1H-indène-1-ylidène[bis(i-butylphoban)]ruthénium (II),
M14 = dichloro[1,3-bis(2-méthylphényl)-2-imidazolindinylidène] (benzylidène) (tricyclohexyl phosphine)ruthénium (II),
M15 = dichloro[1,3-bis(2-méthylphényl)-2-imidazolindinylidène] (2-isopropoxyphénylméthylène)ruthénium (II).

9. Procédé selon la revendication 6, **caractérisé en ce qu'**on utilise, comme catalyseur à base de métal de transition de l'élément ruthénium, au moins M5.

10. Procédé selon l'une quelconque des revendications 1 à 7, **caractérisé en ce que** la quantité dudit au moins un catalyseur dans le mélange réactionnel se situe dans la plage de 0,01 à 10.000% en mole, calculés en tant que somme de tous les catalyseurs utilisés et par rapport à la quantité d'éthène utilisée.

11. Procédé selon l'une quelconque des revendications 1 à 8, **caractérisé en ce qu'**on le réalise par lots.

12. Utilisation d'au moins un catalyseur homogène à base de métal de transition de l'élément ruthénium pour la préparation de 1,3-butadiène à partir d'éthène et d'éthyne.
